Europäisches Patentamt

European Patent Office

Office européen des brevets

(19)

(11) Veröffentlichungsnummer: **0 159 377**
A1

(12) **EUROPÄISCHE PATENTANMELDUNG**

(21) Anmeldenummer: 84104586.7

(51) Int. Cl.⁴: **B 01 D 39/14**

(22) Anmeldetag: 24.04.84

(43) Veröffentlichungstag der Anmeldung: **30.10.85**
**Patentblatt 85/44**

(84) Benannte Vertragsstaaten: **AT CH DE FR GB IT LI NL**

(71) Anmelder: **Carl Schleicher & Schüll GmbH & Co. KG,
Grimsehlstrasse 23, D-3352 Einbeck (DE)**

(72) Erfinder: **Hein, Wolfgang, Dr., Beethovenstrasse 10,
D-3354 Dassel (DE)**

(74) Vertreter: **Jaeger, Klaus, Dr. et al, JAEGER & PARTNER
Patentanwälte Bergstrasse 48 1/2,
D-8035 München-Gauting (DE)**

(54) **Filter und dessen Verwendung.**

(57) Ein Filter mit der von der Trübe zum Filtrat gesehen kennzeichnenden Schichtenfolge Aktivkohlefiltrierpapier (3, 4)/Filtrierkarton (5)/80–600 nm-Polymermembran (6) hält Viren und Pyrogene zurück. Die Filtration erfolgt bei einem Überdruck im Bereich von 0,8 bis 1,5 bar. Bei diesem Druck wird eine Durchflußleistung erzielt, die wesentlich größer als die in der Ultrafiltration erzielbare Durchflußleistung bei drei- bis vierfach höherem Filtrationsdruck ist.

EP 0 159 377 A1

**JAEGER & PARTNER**

PATENTANWÄLTE

24. April 1984

0159377

SLS-107-EP

Jae/eg

Schleicher & Schuell GmbH
Grimsehlstr. 23
3352 Einbeck

---

Filter und dessen Verwendung

---

B e s c h r e i b u n g

Die Erfindung betrifft einen Filter, insbesondere Virenfilter, der im Oberbegriff des Anspruchs 1 genannten Art.

Die Erfindung betrifft weiterhin die Verwendung solcher
Filter.

Zum Abtrennen von Viren, die Abmessungen im Bereich von
ungefähr 10 bis 80 nm aufweisen, insbesondere zum Abtrennen
von Viren aus kontaminierten Wässern, speziell aus kontaminiertem Oberflächenwasser zum Zwecke der Trinkwasseraufbereitung, wurden lange Zeit mit Erfolg Filter aus Asbestfasern verwendet.

Nach dem Erkennen der von Asbestfasern ausgehenden Gesundheitsgefährdung und dem daraus resultierenden Verbot der Verwendung von Asbestfaserfiltern zur Virenfiltration und zum Aufbereiten von Trinkwasser wurde intensiv nach einem Ersatzmaterial für eine wirksame Virenfiltration gesucht.

Da die Suche nach einem praktischen Anforderungen genügenden Filter zur Virenfiltration bislang erfolglos geblieben ist, werden Viren heute auf dem Wege der Ultrafiltration abgetrennt. Die zu reinigende Flüssigkeit wird mit einem Überdruck im Bereich von 5 bar durch eine Polymermembran, also ein Siebfilter, gedrückt, die einen mittleren Porendurchmesser im Bereich von 5 bis 15 nm aufweist. Durch eine solche Ultrafiltration können Viren und Coliphagen, jedoch kaum Pyrogene, das heißt enterotoxische Substanzen, zurückgehalten werden. Der eigentliche Nachteil dieses Verfahrens zum Abtrennen von Viren liegt jedoch in dem Erfordernis der hohen aufzuwendenden Überdrücke und der selbst bei solchem Überdruck nur erzielbaren geringen Durchflußleistung. Zur Not-Trinkwasseraufbereitung, beispielsweise im militärischen Bereich oder im Expeditionsbereich, ist die Ultrafiltration daher ein nur wenig geeignetes Mittel zum Abtrennen von Viren.

Angesichts dieses Standes der Technik liegt der Erfindung die Aufgabe zugrunde, ein Filter, insbesondere für die Trinkwasseraufbereitung, zu schaffen, das bei Betrieb mit geringem Überdruck große Durchflußleistungen ermöglicht und zuverlässig Coliphagen, Viren und Pyrogene zurückhält.

Zur Lösung dieser Aufgabe schafft die Erfindung ein Filter der eingangs genannten Art, das die im Anspruch 1 genannte Schichtenfolge aufweist.

Die Erfindung ist also wesentlich gekennzeichnet durch die Kombination von drei Filterschichten, nämlich (a) einer ersten Schicht aus einem Aktivkohle enthaltenden Filtrierpapier (im folgenden kurz "Aktivkohlepapier" genannt),
(b) einem Filtrierkarton, also einem Faser-Tiefenfilter
ohne jegliche Aktivierung, und (c) einer Polymermembran
mit einer Porengröße, die größer als die zur Siebfiltration
von Viren erforderliche Porengröße ist. Dabei liegt das
Aktivkohlepapier auf der Seite der Trübe, während die Polymermembran auf der Filtratseite des Filters angeordnet
ist. Der Filtrierkarton ist zwischen beiden Schichten eingefügt. Dieses Schichtenfolge und ihre Anordnung, bezogen
auf die Filtrationsrichtung, das heißt die Flußrichtung des
zu behandelnden Mediums während der Filtration, sind wesentliche Elemente der Erfindung.

Vorzugsweise enthält das Filter mindestens zwei, insbesondere
zwei oder drei aufeinanderfolgende Aktivkohlepapiere.

Weiterhin ist es zweckmäßig, als zusätzliche erste Schicht
auf dem oder dem ersten Aktivkohlepapier und als zusätzliche
letzte Schicht unter der Polymermembran jeweils eine Lage
Trenngaze anzuordnen, um dem Filter eine zusätzliche mechanische Festigkeit und Unverletzlichkeit zu verleihen. Die
Trenngaze ist vorzugsweise aus Polyestermonofilen hergestellt.

Die Polymermembran weist üblicherweise eine Porengröße im
Bereich von 80 bis ungefähr 600 nm auf. Wenn die Porengröße
zu klein wird, muß der Filtrationsdruck erhöht werden und
vermindert sich die Durchflußleistung. Wenn die Porengröße
zu groß wird, insbesondere größer als ungefähr 600 nm wird,
gelangen Viren, vermutlich zusammen mit aus dem Aktivkohlepapier ausgeschwemmten Aktivkohleteilchen, und Bakterien
in das Filtrat. Die Porengröße der Polymermembran wird
daher vorzugsweise im Bereich von 150 bis 250 nm, insbesondere
im Bereich um 200 nm, speziell im Bereich von 180 bis 215 nm, möglichst nicht größer als 200 nm, liegen. Diese Angaben
beziehen sich auf mittlere Porengrößen. Insbesondere werden
vorzugsweise Polymermembranen eingesetzt, deren Porengrößenverteilung einen oberen Grenzwert von ungefähr 200 nm bei
einer mittleren Porengröße im Bereich von 180 bis 190 nm
aufweisen. Bei diesen Porengrößen werden optimale Durchflußleistungen bei außerordentlich geringen Filtrationsdrücken und zuverlässiger Virenzurückhaltung erzielt. Selbstverständlich ist ein solches Filter auch gegen Bakterien
undurchlässig. Zudem hält dieses Filter auch Phagen aller
Art, insbesondere Coliphagen, und Pyrogene zurück.

Die tatsächliche synergistische Funktion dieses Filters ist
bislang nicht sicher erklärbar. Wahrscheinlich ist, daß
die Viren zum größeren Teil im Aktivkohle-Tiefenfilter und
zum kleineren Teil im nachgeschalteten Filterkarton zurückgehalten werden, wobei das dem Karton nachgeschaltete
Polymermembranfilter sowohl dem Zurückhalten von Bakterien
als auch dem Zurückhalten gegebenenfalls ausgeschwemmter
Aktivkohleteilchen dient. Diese mögliche Deutung des
Filtrationsvorganges ist jedoch nicht erfindungswesentlich
und erhebt keinen Anspruch auf abschließende Richtigkeit,
da insbesondere der synergistische Einfluß der zwischengeschalteten Filterkartonschicht, die erfindungswesentlich ist,
bislang noch nicht restlos erklärt werden kann. Gesichert
ist derzeit lediglich, daß diese Schicht offensichtlich
eine wesentliche Rolle bei der Virenrückhaltung spielt.

Nach einer weiteren Ausgestaltung der Erfindung liegen die
vorzugsweise aus Cellulosenitrat bestehende Polymermembran
und der vorzugsweise aus Linters-Karton bestehende Filtrierkarton als einheitliches integriertes Flächenverbundmaterial,
vor. Solche Verbundstrukturen sind    als kartongestützte
Polymermembranen      an sich bekannt und dienen zur
Bakterienfiltration. Sie sind alleine nicht in der Lage, Viren zurückzuhalten.

Das Aktivkohle enthaltende Filtrierpapier ist vorzugsweise ein Filtrierpapier auf Cellulosefaserbasis und ist in an sich bekannter Weise nach üblichen Verfahren homogen mit feinstverteilter Aktivkohle beladen. "Beladen" heißt dabei, daß die Aktivkohle der der Herstellung des Filtrierpapiers dienenden Cellulosefaseraufschwemmung in homogener Verteilung zugemischt wird. Dieses Aktivkohlepapier sollte im Hinblick auf seine Wirksamkeit nicht weniger als ungefähr 5 bis 10 Gew.-% Aktivkohle enthalten und sollte im Hinblick auf ein Ausschwemmen der Aktivkohle nicht mehr als ungefähr 50 Gew.-% Aktivkohle enthalten. Optimale Ergebnisse werden für ein Aktivkohlepapier erhalten, das, bezogen auf das Gesamtgewicht des Filtrierpapiers, 15 bis 30 Gew.-% Aktivkohlepulver enthält. Die spezifische Oberfläche der zur Herstellung des Aktivkohlepapiers verwendeten Aktivkohle liegt vorzugsweise im Bereich zwischen 700 und 1500 m²/g , insbesondere im Bereich von 900 bis 1300 m²/g. Von Aktivkohlepapieren dieser Art ist beispielsweise aus der deutschen Offenlegungsschrift DE 24 03 971 A1 bekannt, daß sie selbst in Gegenwart von bakteriziden Metallen oder Metallverbindungen im Papier nur Bakterien bis zu einer Größer von 40 nm, nicht aber Viren oder Pyrogene zurückzuhalten vermögen. Daß ein solches Aktivkohlepapier ohne zusätzliche bakterizide Aktivierung in Verbindung mit einem fasrigen Tiefenfilterkarton und einer relativ großporigen Polymermembran in der Lage ist, Viren praktisch 100%ig zurückzuhalten, ist überraschend und bislang nicht sicher erklärbar. Ausgedehnte Versuche haben jedoch gezeigt, daß mit dem Filter gemäß der Erfindung aus einer Viren, Coliphagen und Pyrogene enthaltenden Trübe bei Aufwendung eines Filtrationsüberdruckes von 1 bar innerhalb 1 h mindestens zwei Liter Trinkwasser hergestellt werden können, das frei von Viren, Phagen und insbesondere auch frei von Pyrogenen ist. Aufgrund dieser Eigenschaften eignet sich das Filter gemäß der Erfindung insbesondere
zur Not-Trinkwasseraufbereitung aus Oberflächenwasser
unbekannter Beschaffenheit, insbesondere im militärischen
Bereich und im Expeditionsbereich. Vor allem für diese
Verwendungszwecke wird das Filter gemäß der Erfindung
vorzugsweise als Flachfilterzuschnitt, insbesondere als
Rundfilter, eingesetzt, bei denen die einzelnen Schichten
dann rund um den Außenrand herum fest miteinander verbunden
sind, beispielsweise durch Verkleben, Verschweißen, Verpressen oder insbesondere prägendes Verpressen.

Die Erfindung ist im folgenden anhand von Ausführungsbeispielen in Verbindung mit der Zeichnung näher erläutert.
Dabei zeigt die einzige Figur, nämlich die

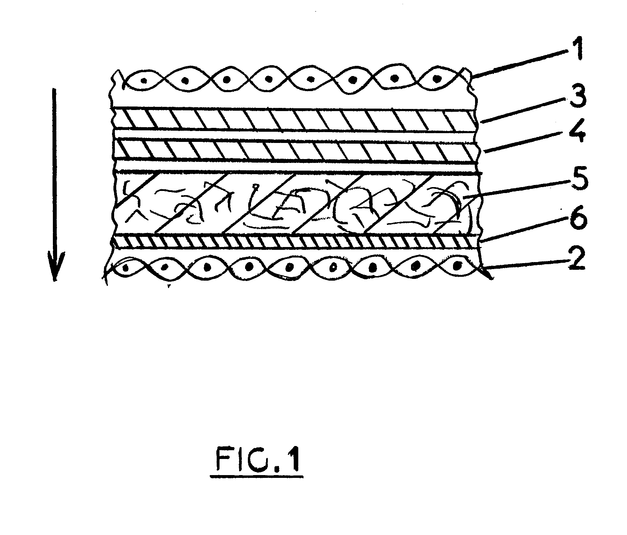

Fig. 1      in schematischer Darstellung das Filter
            im Querschnitt.

In der Fig.1 ist in schematischer Darstellung ein Schnitt
durch ein Filter gemäß der Erfindung gezeigt, wobei der
im linken Teil der Zeichnung wiedergegebene Pfeil die Filtrationsrichtung andeutet, in der Darstellung der Fig. 1
also die zur Trübe gewandte Seite des Filters oben und die
zum Filtrat gewandte Seite des Filters unten dargestellt
ist.

In der aus Fig. 1 ersichtlichen Weise ist das Filter mit
seinen verschiedenen Schichten zwischen je eine Lage
Trenngase 1,2 sandwichartig eingeschlossen. Die Trenngaze
bestehen aus Polyestermonofilen.

Nach der obersten Lage der Trenngaze 1 folgen zwei identische Cellulosefaser-Filtrierpapiere 3,4 , die jeweils
25 Gew.-% Aktivkohle pflanzlicher Herkunft mit einer
spezifischen Oberfläche von 1200 m²/g homogen in der Fasermatrix verteilt enthalten. Die beiden Aktivkohlepapiere
3,4 sind nicht speziell miteinander flächig oder punktuell
verbunden, sondern sind lediglich im Rahmen des Gesamtverbundes außerhalb der für die Filtration benutzten freien
Fläche um ihren gesamten Außenrand miteinander verbunden.

Den beiden Aktivkohlepapierschichten 3,4 ist eine Flächenverbundstruktur nachgeschaltet, die aus einem vergleichsweise starken Linters-Filtrierkarton (ca. 300 g/m²) 5 und
einer unmittelbar auf dessen Oberfläche aufgebrachten Polymermembran 6 aus Cellulosenitrat besteht. Dabei ist diese
Flächenverbundstruktur 5,6 so in den Verband des Filters
eingeordnet, daß der Filtrierkarton 5 der Trübenseite
und den Aktivkohlepapieren 3,4 zugewendet ist, also stromauf, bezogen auf die Filtrationsrichtung, liegt, während
die Polymermembran der unteren Trenngaze 2 bzw. dem Filtratraum zugekehrt ist, bezüglich der Kartonschicht 5 also
stromab liegt. Die Polymermembran 6 hat eine Nennporengröße
von 200 nm mit größten Porenöffnungen von kleiner als höchstens
220 nm.

Das in der Fig. 1 gezeigte Filtermaterial wird kontinuierlich
in Bahnen durch aufeinanderfolgendes  Aufeinanderführen der
einzelnen Schichtmaterialien hergestellt. Aus der so hergestellten Bahn werden für die nachstehend wiedergegebenen
Versuche Rundfilter mit einem Durchmesser von 50 mm gestanzt.
Gleichzeitig mit dem Stanzvorgang werden die Filterschichten
auf einer Breite von ungefähr 1 mm um ihren Außenrand herum
durch Prägen  mit einem Prägekranz so ausreichend fest miteinander verbunden, daß das Filter mit allen seinen Schichten
als integrierte Einheit handhabbar ist.

Versuch 1
_____

500 ml Leitungswasser werden mit einem Coliphagen infiziert,
der eine Größe von ca. 25 bis 30 nm aufweist. Das kontaminierte Wasser wird bei Raumtemperatur mit einem Druck von
1 bar durch das vorstehend beschriebene Filter filtriert.
Durch die Filtration konnten die Plaquezahlen der Coliphagen
um den Faktor $10^3$ reduziert werden.

Versuch 2
_____

15 l Leitungswasser werden mit $5 \cdot 10^4$ bac. globigii-Sporen
je ml Wasser kontaminiert. Die 15 l kontaminiertes Wasser
werden bei einem Filtrationsdruck von 1 bar kontinuierlich
über ein und denselben Filter filtriert. Die Analyse des
Filtrats zeigt, daß die Sporen zu 100% zurückgehalten werden.

Versuch 3
_____

500 ml Leitungswasser werden mit Staphylokokkenenterotoxin
Typ B in einer Konzentration von 1 µg/ml kontaminiert. Die
kontaminierte Trübe wird mittels einer handbeschaufschlagten
Luftpumpe bei einem Druck von ungefähr 0,8 bar durch das
in der vorstehend beschriebenen Weise hergestellte Rundfilter
filtriert.

Die Analyse des Filtrats zeigt, daß das Pyrogen zu 100%
zurückgehalten wird.

Versuch 4
_____

Unter Verwendung von pyrogenfreiem destilliertem Wasser
werden 100 ml einer Lösung hergestellt, die 0,02 µg/ml einer
im Handel unter der Bezeichnung Pyrexal® erhältlichen pyrogenhaltigen Lösung enthalten.

50 ml dieses Ansatzes werden durch den vorstehend beschriebenen Rundfilter bei einem Druck von 1,2 bar filtriert.
Drei Versuchstiere (Kaninchen) werden mit jeweils 10 ml
unfiltrierter Lösung unter Zusatz von 1 ml 8,5 %iger
Kochsalzlösung intravenös gespritzt, während weitere drei
Versuchstiere mit jeweils 10 ml filtrierter Lösung unter
Zusatz von ebenfalls 1 ml 8,5 %iger Kochsalzlösung
in gleicher Weise gespritzt werden. Der Temperaturanstieg der
Tiere wird vor Versuchsbeginn und drei Stunden nach Injektion
gemessen. Der Temperaturanstieg bei den Tieren, denen die
unfiltrierte Lösung injiziert worden ist, beträgt im Mittel
1,6° C. Der mittlere Temperaturanstieg bei den Tieren,
denen die filtirierte Lösung injiziert worden ist, beträgt
dagegen lediglich 0,3° C. Dies Ergebnis zeigt, daß das
Filter gemäß der Erfindung selbst Pyrogene wirksam zurückhält.

**JAEGER & PARTNER**

PATENTANWÄLTE

24. April 1984

0159377

SLS-107-EP

Jae/eg

Schleicher & Schuell GmbH

Grimsehlstr. 23

3352 Einbeck

Filter und dessen Verwendung

A n s p r ü c h e

1. Filter, insbesondere Virenfilter,
   g e k e n n z e i c h n e t  durch
   die folgende, in Richtung von der Trübe zum Filtrat
   gesehene Kombination und Folge von Schichten:

   a) Aktivkohle enthaltendes Filtrierpapier (3,4);

   b) Filtrierkarton (5) und

   c) eine Polymermembran (6) mit einer Porengröße im
   Bereich von 80 bis 600 nm.

2. Filter nach Anspruch 1,
dadurch  g e k e n n z e i c h n e t,
daß die mittlere Porengröße der Polymermembran (6)
im Bereich von 150 bis 250 nm, insbesondere im Bereich
von 180 bis 215 nm liegt.

3. Filter nach einem der Ansprüche 1 oder 2,
dadurch  g e k e n n z e i c h n e t,
daß der als Schicht (b) verwendete Filtrierkarton (5)
und die Polymermembran (6) als einheitliches Flächenverbundmaterial vorliegen.

4. Filter nach einem der Ansprüche 1 bis 3,
dadurch  g e k e n n z e i c h n e t,
daß die Schicht (a) (3,4) ein Filtrierpapier auf
Cellulosefaserbasis ist, das 15 bis 30 Gew.-%
Aktivkohlepulver enthält.

5. Filter nach einem der Ansprüche 1 bis 4,
dadurch  g e k e n n z e i c h n e t,
daß der Filtrierkarton (5) ein Linterskarton ist
und die Polymermembran (6) zumindest im wesentlichen
aus Cellulosenitrat besteht.

6. Filter nach einem der Ansprüche 1 bis 5,
g e k e n n z e i c h n e t  durch
mindestens zwei unmittelbar vor dem Filtrierkarton
(5) aufeinanderfolgende Schichten von Aktivkohle enthaltendem Filtrierpapier (3,4).

7. Filter nach einem der Ansprüche 1 bis 6,
dadurch  g e k e n n z e i c h n e t,
daß über der oder über der ersten Aktivkohle enthaltenden Filtrierpapierschicht (3) und unter der Polymermembran (6) jeweils eine Lage Trenngaze (1,2) aus Polyestermonofilen angeordnet ist.

8. Filter nach einem der Ansprüche 1 bis 7,
   dadurch   g e k e n n z e i c h n e t,
   daß das Filterelement als Flachfilterzuschnitt ausgebildet ist und alle Schichten (1,2,3,4,5,6) des Filters
   einschließlich der Trenngaze (1,2), soweit vorhanden,
   außerhalb der aktiven Filterfläche umlaufend fest miteinander verbunden sind, speziell durch Klebe-, Schweiß-,
   Press-, oder Prägeverbund.

9. Verwendung des Filters nach einem der Ansprüche
   1 bis 8,
   zum Abtrennen von Viren,Coliphagen und Pyrogenen, insbesondere aus kontaminiertem Oberflächenwasser.

FIG.1

Europäisches
Patentamt

EUROPÄISCHER RECHERCHENBERICHT

0159377
Nummer der Anmeldung

EP 84 10 4586

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CH-A- 556 682 (GHH BASEL)  <br><br> * Hauptansprüche 1, 2; Unteranspruch 2; Spalte 1, Zeile 1 - Spalte 2, Zeile 15; Spalte 3, Zeilen 3-30; Spalte 5, Zeilen 15-26, 34-44 * <br><br> --- | 1-5,7-9 | B 01 D 39/14 |
| A | DE-U-8 228 725 (SEITZ-FILTER-WERKE) <br> * Ansprüche 1, 2; Seite 8, Zeile 16 - Seite 9, Zeile 15; Figur 2 * <br><br> ----- | 1,3,8 | |

RECHERCHIERTE SACHGEBIETE (Int. Cl.4)

B 01 D 13/04
B 01 D 37/00
B 01 D 39/00
B 01 D 39/14
C 02 F 1/44
C 12 M 3/00

Der vorliegende Recherchenbericht wurde für alle Patentansprüche erstellt.

| Recherchenort <br> BERLIN | Abschlußdatum der Recherche <br> 14-12-1984 | Prüfer <br> KUEHN P |
|---|---|---|